# EUROPEAN PATENT SPECIFICATION

(11) **EP 3 505 153 B1**
(45) Date of publication and mention of the grant of the patent: **24.03.2021**
(21) Application number: 17020599.1
(22) Date of filing: 31.12.2017
(51) Int. Cl.: A61P 11/12

(54) **A PHARMACEUTICAL COMPOSITION COMPRISING ACETYLCYSTEINE WHICH IS FREE OF METHYLPARABEN AND PROPYLPARABEN**
PHARMAZEUTISCHE ZUSAMMENSETZUNG MIT ACETYLCYSTEIN, DIE FREI VON METHYLPARABEN UND PROPYLPARABEN IST
COMPOSITION PHARMACEUTIQUE COMPRENANT DE L'ACÉTYLCYSTÉINE, QUI EST EXEMPT DE MÉTHYLPARABÈNE ET DE PROPYLPARABÈNE

(43) Date of publication of application: 03.07.2019
(73) Proprietor: Abdi Ibrahim Ilac Sanayi ve Ticaret A.S., 34500 Esenyurt/Istanbul (TR)
(72) Inventor: Sarikaya, Ali Osman, Esenyurt- Istanbul (TR); Tanriver, Koray, Esenyurt- Istanbul (TR); Kumar, Udaya Dude, Esenyurt- Istanbul (TR); Akdemir, Emine, Esenyurt- Istanbul (TR); Kayar, Gul Gonul, Esenyurt- Istanbul (TR)

(56) References cited:
- EP-A1- 0 340 662
- GB-A- 2 192 789
- TR-A2- 200 703 131

## Description

### Technical Field of Invention

The present invention relates to obtaining stability and smell improved pharmaceutical composition comprising acetylcysteine and pharmaceutically acceptable excipients which is free of methyl paraben and propyl paraben.

### Background of the Invention

Acetylcysteine is N acetyl derivative of L-cysteine. Its chemical name is (R)-2-acetamido-3-sulfanyl propionic acid and its chemal structure is shown in Formula 1.

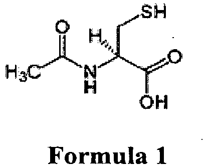

The structure of the sulfhydryl group has a mucolytic effect on mucoid and mucopurulent secretions due to its ability to break disulfide bonds in the glycoprotein.

It is also used as a cough treatments because it facilitates the removal of mucus by reducing the accumulation of sputum accumulated in the airways. This makes acetylcysteine useful for decreasing abnormal dense mucus by breaking disulphide bonds in patients with cystic fibrosis.

The impurities of N-acetylcysteine that are specified in European Pharmacopea are:

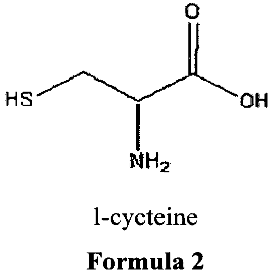

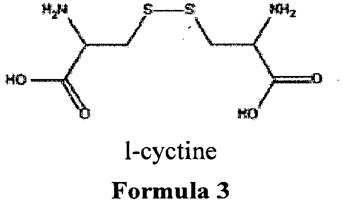

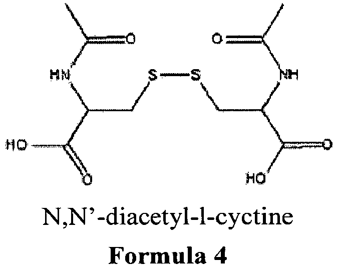

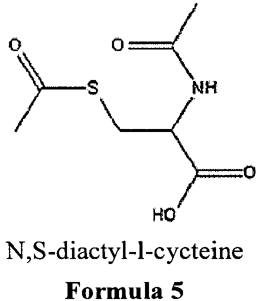

TR 2007/03131 relates to dosage forms comprising 900 mg to 1200 mg N-acetylcysteine.

GB 2192789 discloses pharmaceutical compositions comprising water soluble effervescent granules. Acetylcysteine, aspartam, sorbitol and orange as sweetener are mixed.

EP 0340662 discloses water soluble solid pharmaceutical composition comprising acetylcysteine, xylitol, sodium saccharine dihydrate beta carotene and sweeteners.

Various formulation strategies have been improved for acetylcysteine as stated above. This is due to the fact that acetylcysteine is an unstable molecule and the pharmaceutical composition tends to be degrade even at the time of preparation. In addition to formation of some impurities in the storage step, problems such as formation of malodorous sulfur-containing compounds like H2S are seen. This stability problems limit the acetylcysteine formulation. Especially bad smell causes the patients to give up the treatment, and complete recovery is not achieved.

Furthermore, parabens are one of the most used preservatives in drugs. However, a growing number of formulations tend to not use parabens due to possible safety problems. Even though it is not proved, it is thougt that parabens imitate osterogen and they are related to breast cancer. Therefore, alternative formulations free of paraben are initiated to be improved.

Another problem encountered in the production of drugs is the decrease in flowability and the observation of agglomeration during filling.

The present invention relates to obtaining stability and smell improved pharmaceutical composition comprising acetylcysteine and pharmaceutically acceptable excipients which is free of methyl paraben and propyl paraben that solves the aforementioned technical problems.

### Description of the Invention

The present invention relates to obtaining stability and smell improved pharmaceutical composition comprising acetylcysteine and pharmaceutically acceptable excipients which is free of methyl paraben and propyl paraben.

Pharmaceutical dosage form of the present invention can be in granule, tablet (core, film coated, effervescent, orally disintegrating, water soluble), capsule, sachet, pastil, syrup, suspension, solution and solution and is not limited to thereto. Preferably, it is in granule form.

Another objection of the invention is to obtain a stability and smell improved product by dry mixing without any process.

Another objection of the invention is to provide decrease in agglomeration and increase in flowability.

Another objection of the invention is to obtain a masking formulation so that unpleasant taste and odor of acetylcysteine is not felt when it is both granule and reconstituted.

In preferred embodiment of the invention, the mean particule size(d50) of acetylcysteine is between 1 µm and 100 µm, preferably between 50 µm and 85 µm. The value of d90 is between 1 µm and 200 µm, preferably between 1 µm and 185 µm. Particule size can be determined by laser light scattering (laser diffraction) by Mastersizer 2000 instrument. The term "d90 particle size" used herein refers to the particle size of 90% of particles by volume and the term "d50 particle size" refers to the particle size that 50 of particles have in volume.

Another objection of the invention is to obtain a formulation wihout methyl paraben and propyl paraben different from existing products in the market.

In a preferred embodiment of the invention, benzoic acid is used in micronized form. Thus, homogenous distribution of granules in the formulation is provided. In addition, although benzoic acid is slighty soluble in cold water and acid medium, the inventors obtained stability and smell improved product which is formulated by using micronized benzoic acid with sodium EDTA and which is homogeneous solution at low pH when reconstituted.

In a preferred embodiment of the invention, pharmaceutical composition can be selected among tablet, granule, sachet and capsule solid oral dosage forms. Preferably, composition is in granule dosage form.

The term "stability" used herein refers to stability that is followed at different tmeperatures and relative humidities (RH) (25 °C 60% RH, 30 °C 65% RH, 40 °C 75% RH) and /or in-use stability (1^{st} and 12^{th} days). As the product is administered by reconstituting, in-use stability is important in terms taste, smell and patient convenience.

The term "paraben" refers to methyl paraben and propyl paraben.

In a preferred embodiment of the invention, excipients comprise chelating agent, antimicrobial preservative, colorant, sweetener and flavorant and it is not limited thereto.

In a preffered embodiment of the invention, chelating agent can be at least one selected from disodium edetate, pentetic acid, citric acid monohydrate, edetate calcium disodium, edetic acid, fumaric acid, malic acid, maltol, trisodium edetate or mixtures thereof and is not limited thereto. Preferebly, it is disodium edetate. Disodium edetate can also be called disodium EDTA, EDTA disodium.

Same amount of excipients with different concentrations of disodium EDTA were tried for present invention and the optimum amount of disodium EDTA was determined in terms of in-use stability. Table 1 summarizes the trials. Trial D1 comprises 0.005% disodium EDTA by weight of the total weight of the pharmaceutical composition, D2 comprises 0.025 % disodium EDTA by weight of the total weight of the pharmaceutical composition and D3 comprises 0.05 % disodium EDTA by weight of the total weight of the pharmaceutical composition.

**TABLE 1 - In-use stability results at different disodium edetate concentrations.**

| **Impurities** | **D1** | **D2** | **D3** |
|---|---|---|---|
| Imp A* | Not dedected | Not dedected | Not dedected |
| Imp B* | 0.605 | 0.390 | 0.38 |
| Imp C* | 0.348 | 0.180 | 0.170 |
| Imp D* | 0.547 | 0.460 | 0.420 |
| Total unknown impurity | Not dedected | Not dedected | Not dedected |

| | | | |
|---|---|---|---|
| **Imp A(L-cystine): 3,3'-disulfanediylbis[(2R)-2-aminopropanoic acid],* *Imp B(L-cysteine): (2R)-2-amino-3-sulfanylpropanoic acid* *Imp C(N,N'-diacetyl-l-cystine): (2R,2'R)-3,3'-disulfanediylbis[2-(acetylamino)propanoic acid]* *Imp D(N,S-diacetyl-l-cysteine):(2R)-2-(acetylamino)-3-(acetylsulfanyl)propanoic acid* | | | |

As a result of the data obtained from Table 1, it was determined that disodium EDTA should be in 0.01% to 0.1% by weight, preferably 0.025% to 0.05% by weight of total weight of the pharmaceutical composition in the present invention.

In a preferred embodiment of the present invention, sweetener can be at least one selected from sucrose, sucralose, aspartame, saccharine, acesulfam K, cyclamates, glucose, fructose, lactose and other sugars, xylitol, erythritol, sorbitol, mannitol and other sugar alcohols or mixtures thereof and is not limited thereto. Preferably, it is sucrose. Composition comprises 10% to 90%, preferably 60% to 90% sweetener by weight of total weight of the pharmaceutical composition in the present invention.

In a preferred embodiment of the present invention, flavorant can be at least one selected from fruit flavors such as orange, banana, strawberry, cherry, lemon, mentol, mint, cinnamon and flavorants thereof or mixtures thereof and is not limited thereto. Preferably, it is orange flavor.

In a preferred embodiment of the present invention, colorant can be at least one selected form carotenoid, titanium dioxide, iron oxide, tartrazine, riboflavin, brilliant blue, anthocyanins or mixtures thereof and is not limited thereto. Preferably, it is carotenoid, more preferably, beta-caroten.

In a preferred embodiment of the present invention, antimicrobial agent can be at least one selected from benzoic acid, benzalkonium chloride, benzyl alcohol, calcium acetate, cresol, cetylpyridinium chloride or mixtures thereof and is not limited thereto. Preferably, it is benzoic acid.

Table-2 below shows results of in-use stability of formulations comprising paraben and benzoic acid. D4 represents paraben comprising product and D5 represents benzoic acid comprising product.

**Table-2-In-use stability of formulations comprising paraben and benzoic acid.**

| **Impurities** | **D4** | **D5** |
|---|---|---|
| Imp A* | Not dedected | Not dedected |
| Imp B* | 0.64 | 0.43 |
| Imp C* | 0.48 | 0.24 |
| Imp D* | 0.45 | 0.36 |
| Total Unknown impurity | 0.02 | 0.07 |

It is observed that benzoic acid comprising product is more stable than paraben comprising product.

Preferably micronized benzoic acid is used in the present invention wherein mean particle size (d50) of it is between 1 µm and 20 µm, preferably between 10 µm and 20 µm. The value d90 is between 10 µm and 50 µm, preferably between 30 µm and 50 µm.

In one embodiment of the present invention, composition comprises acetylcysteine, micronized benzoic acid, disodium EDTA and at least one pharmaceutically acceptable excipient wherein mean particle size (d50) of benzoic acid and acetylcysteine is between 1 µm and 20 µm and between 1 µm and 100 µm, respectively and comprises 0.01% to 0.1% disodium EDTA by weight.

In one embodiment of the present invention, sweetener is used in portions during the prepation of the composition. Thus, a method for preparing pharmaceutical composition of the present invention comprises the following steps:
a) sieving a portion of sweetener
b) sieving micronized benzoic acid, disodium acetate seperately, adding them into sweetener in step (a) and mixing and again sieving and mixing.
c) sieving another portion of sweetener and adding to mixture in step (b)
d) sieving acetylcysteine and another portion of the sweetener and adding to mixture in step (c)
e) sieving the rest of the sweetener and adding to mixture in step (d) and mixing
f) bringing the final mixture to desired dosage form.
In this method, sieving process is carried out by using between 0.5 mm and 1.2 mm screen.

In a preferred embodiment of the present invention, a method for preparing a granule pharmaceutical composition comprising benzoic acid having mean particle size of 1 µm to 20 µm, acetylcysteine having mean particle size of 1 µm - 100 µm and 0.01% to 0.1% disodium EDTA by weight of the total weight of the composition comprises the following steps:
a) sieving a portion of sweetener
b) sieving micronized benzoic acid, disodium acetate seperately, adding them into sweetener in step (a) and mixing and again sieving and mixing.
c) sieving another portion of sweetener and adding to mixture in step (b)
d) sieving acetylcysteine and another portion of the sweetener and adding to mixture in step (c)
e) sieving the rest of the sweetener and adding to mixture in step (d) and mixing
f) filling.

The present invention is further defined by reference to the following example.

### Example:

| **Ingredient** | **%** |
|---|---|
| | **(g/40g)** |
| Acetylcysteine | 10% - 11% |
| Sucrose | 80% - 90% |
| Benzoic acid(micronized) | 0.1% - 0.2% |
| Beta-carotone | 0.0025% - 0.1% |
| Orange flavor | 2.5% - 5% |
| Disodium edetate | 0.05% |
| **Granule Weight** | 100 |

### Production method:

a) sieving half of sucrose.
b) sieving micronized benzoic acid, disodium acetate, beta-carotene and orange flavor seperately, adding them into sucrose in step (a) and mixing and again sieving and mixing.
c) sieving a portion of rest of sucrose and adding to mixture in step (b)
d) sieving acetylcysteine and another portion of rest of sucrose and adding to mixture in step (c)
e) sieving the rest of the sucrose and adding to mixture in step (d) and mixing
f) filling.

## Claims

1. Stability and smell improved pharmaceutical composition comprising acetylcysteine wherein it comprises micronized benzoic acid, disodium EDTA and at least one pharmaceutically acceptable excipient and does not comprise methyl paraben and propyl paraben.

2. A pharmaceutical composition according to claim 1, wherein mean particle size (d50) of acetycysteine is between 1 µm - 100 µm, preferably 50µm - 85 µm.

3. A pharmaceutical composition according to claim 1, wherein particle size (d90) of acetycysteine is between 1 µm -200 µm, preferably 1 µm - 185 µm.

4. A pharmaceutical composition according to claim 1, wherein it comprises chelating agent, antimicrobial preservative, colorant, sweetener and flavorant.

5. A pharmaceutical composition according to claims 1 or 4, wherein chelating agemt is disodium EDTA.

6. A pharmaceutical composition according to claim 1, wherein it comprises 0.01% to 0.1% disodium EDTA by weight of total weight of the composition.

## Patentansprüche

1. Stabilität und Geruch verbesserten die pharmazeutische Zusammensetzung, die Acetylcystein enthielt, wobei sie mikronisierte Benzoesäure, Dinatrium-EDTA und mindestens einen pharmazeutisch verträglichen Hilfsstoff enthielt und kein Methylparaben und Propylparaben enthielt.

2. Pharmazeutische Zusammensetzung nach Anspruch 1, wobei die mittlere Teilchengröße (d50) von Acetylcystein zwischen 1 um - 100 um, vorzugsweise 50 um - 85 um liegt.

3. Pharmazeutische Zusammensetzung nach Anspruch 1, wobei die Partikelgröße (d90) von Acetylcystein zwischen 1 um und 200 um, vorzugsweise 1 um bis 185 um liegt.

4. Pharmazeutische Zusammensetzung nach Anspruch 1, wobei sie Chelatbildner, antimikrobielles Konservierungsmittel, Farbstoff, Süßstoff und Aromastoff enthält.

5. Pharmazeutische Zusammensetzung nach Anspruch 1 oder 4, wobei der Chelatbildner Dinatrium-EDTA ist.

6. Pharmazeutische Zusammensetzung nach Anspruch 1, wobei sie 0,01 bis 0,1 Gew .-% Dinatrium-EDTA des Gesamtgewichts der Zusammensetzung enthält.

## Revendications

1. Composition pharmaceutique à stabilité et odeur améliorées comprenant de l'acétylcystéine; dans lequel il contient de l'acide benzoïque micronisé, de l'EDTA disodique et au moins un excipient pharmaceutiquement acceptable et ne contient pas de méthyl paraben et de propyl paraben.

2. Composition pharmaceutique selon la revendication 1, dans laquelle la taille moyenne des particules (d50) de l'acétylcystéine est comprise entre 1 um - 100 um, de préférence 50um - 85 um.

3. Composition pharmaceutique selon la revendication 1, dans laquelle la taille des particules (d90) d'acétycystéine est comprise entre 1 um -200 um, de préférence 1 um-185 um.

4. Composition pharmaceutique selon la revendication 1, dans laquelle elle contient un agent chélatant, un conservateur antimicrobien, un colorant, un édulcorant et un arôme.

5. Composition pharmaceutique selon les revendications 1 ou 4, dans laquelle l'agent chélatant est l'EDTA disodique.

6. Composition pharmaceutique selon la revendication 1, dans laquelle elle contient 0,01% à 0,1% d'EDTA disodique en poids du poids total de la composition.
